# FASCICULE DE BREVET EUROPEEN

(11) **EP 2 346 491 B1**
(45) Date de publication et mention de la délivrance du brevet: **07.12.2016**
(21) Numéro de dépôt: 09760945.7
(22) Date de dépôt: 16.10.2009
(51) Int. Cl.: A61K 9/08, A61K 31/337, A61K 31/695, A61P 35/00

(54) **NOUVELLES UTILISATIONS PHARMACEUTIQUES DE NANOPARTICULES**
NEUE PHARMAZEUTISCHE VERWENDUNG VON NANOPARTIKELN
NOVEL PHARMACEUTICAL USES FOR NANOPARTICLES

(30) Priorité: 17.10.2008 FR 0857100
(43) Date de publication de la demande: 27.07.2011
(73) Titulaire: Centre National de la Recherche Scientifique (C.N.R.S.), 75016 Paris (FR); Université Claude Bernard Lyon I, 69625 Villeurbanne Cedex (FR); Institut National de la Santé et de la Recherche Médicale, 75013 Paris (FR)
(72) Inventeur: LYSENKO, Volodymyr, F-69100 Villeurbanne (FR); GELOEN, Alain, F-69008 Lyon (FR); MOGNETTI, Barbara, I-10153 Turin (IT)
(74) Mandataire: Lavoix
(86) Numéro de dépôt international: PCT/FR2009/051988
(87) Numéro de publication internationale: WO 2010/043832

(56) Documents cités:
- US-A- 5 593 783
- DATABASE WPI Week 200564 Thomson Scientific, London, GB; AN 2005-622868 XP002517577 & JP 2005 232066 A (APUTO KK) 2 septembre 2005 (2005-09-02) & JP 2005 232066 A (APUTO KK) 2 septembre 2005 (2005-09-02)
- BOTSOA J ET AL: "Application of 3C-SiC quantum dots for living cell imaging" APPLIED PHYSICS LETTERS, AIP, AMERICAN INSTITUTE OF PHYSICS, MELVILLE, NY, vol. 92, no. 17, 2 mai 2008 (2008-05-02), pages 173902-173902, XP012106740 ISSN: 0003-6951 cité dans la demande
- HERLIN-BOIME N ET AL: "Flame Temperature Effect on the Structure of SiC Nanoparticles Grown by Laser Pyrolysis" JOURNAL OF NANOPARTICLE RESEARCH ; AN INTERDISCIPLINARY FORUM FOR NANOSCALE SCIENCE AND TECHNOLOGY, KLUWER ACADEMIC PUBLISHERS, DO, vol. 6, no. 1, 1 février 2004 (2004-02-01), pages 63-70, XP019260115 ISSN: 1572-896X
- DATABASE WPI Week 200404 Thomson Scientific, London, GB; AN 2004-039887 XP002562098 & JP 2003 347212 A (DOKURITSU GYOSEI HOJIN SANGYO GIJUTSU SO) 5 décembre 2003 (2003-12-05)
- S. A. ALEKSEEV; V. N. ZAITSEV; J. BOTSOA; D. BARBIER: "Fourier Transform Infrared Spectroscopy and Temperature-Programmed Desorption Study of Surface Chemistry of Porous 6H-SiC" CHEMISTRY OF MATERIALS, vol. 19, 2007, pages 2189-2194, XP002517576 Washington D.C., USA cité dans la demande
- VAUGHAN GERALD L ET AL: "The toxicity of silicon carbide whiskers: A review" JOURNAL OF ENVIRONMENTAL SCIENCE AND HEALTH, PART A: ENV.SCIENCE AND ENGINEERING, NEW YORK, NY, US, vol. 31, no. 8, 1 janvier 1996 (1996-01-01), pages 2033-2054, XP009112987
- DUFRESNE A ET AL: "Pulmonary retention of ceramic fibers in silicon carbide (SiC) workers" 1 janvier 1995 (1995-01-01), AIHA JOURNAL, TAYLOR & FRANCIS, PAGE(S) 490 - 498 , XP009112986 ISSN: 0002-8894 le document en entier
- EDLING C ET AL: "MORTALITY AND CANCER INCIDENCE AMONG WORKERS IN AN ABRASIVE MANUFACTURING INDUSTRY" BRITISH JOURNAL OF INDUSTRIAL MEDICINE, BRITISH MEDICAL ASSOCIATION, LONDON, GB, vol. 44, no. 1, 1 janvier 1987 (1987-01-01), pages 57-59, XP009112991 ISSN: 0007-1072

## Description

La présente invention a pour objet de nouvelles utilisations pharmaceutiques de nanoparticules de SiC (carbure de silicium) de taille inférieure à 10 nm, notamment dans le domaine du traitement du cancer.

L'élimination des cellules cancéreuses est assurée par chimiothérapie en utilisant des molécules de synthèse principalement anti-mitotiques. Les médicaments antinéoplasiques classiques inhibent la prolifération cellulaire en interagissant de façon non-spécifique avec les processus de réplication cellulaire (duplication des acides nucléiques, fonctionnement correct du fuseau mitotique, synthèse des purines et des pyrimidines, etc.). Les médicaments antinéoplasiques innovants, par contre, sont principalement des anticorps monoclonaux dirigés contre des antigènes tumoraux spécifiques. Ces molécules peuvent être nues (comme, par exemple, le Herceptin^{®}) ou véhiculer des médicaments cytotoxiques ou des particules radioactives (comme, par exemple, le Ibritumomab, administré en association avec d'autres médicaments antinéoplasiques pour traiter les lymphomes non hodgkiniens).

L'utilisation des molécules anti-mitotiques présente un inconvénient majeur. En effet, ces molécules s'attaquent à toutes les cellules cancéreuses mais également aux cellules non cancéreuses. Cela implique que de nombreux types cellulaires à division rapide sont touchés (peau, globules rouges, globules blancs, plaquettes...). Les effets secondaires peuvent donc être très importants et souvent on observe une réduction des schémas thérapeutiques, ce qui limite l'efficacité de la thérapie.

Une partie des médicaments antinéoplasiques classiques, en outre, est cancérogène, ce qui implique un risque de développer une nouvelle tumeur chez les patients ainsi traités, mais aussi chez les personnes préposées à la manipulation des médicaments. Les médicaments de deuxième génération, qui, dans la plupart des cas, sont encore considérés comme traitements de deuxième choix, conjointement à des effets secondaires mineurs, offrent souvent un résultat thérapeutique inférieur à celui des médicaments classiques, et en plus sont extrêmement coûteux. Une utilisation pertinente figure dans le document JP2005232066A.

Ainsi, la présente invention a pour but de fournir un nouveau médicament anti-cancéreux sélectif et efficace, c'est-à-dire un médicament efficace sur les cellules cancéreuses mais n'attaquant pas les cellules non cancéreuses, et dont, en plus, la production est relativement peu onéreuse.

La présente invention a pour but de fournir un médicament anti-cancéreux inhibant la survie cellulaire des cellules cancéreuses sans inhiber celle des cellules non cancéreuses.

La présente invention concerne des nanoparticules de SiC de taille inférieure à 10 nm pour leur utilisation dans le cadre du traitement du cancer.

La présente invention concerne donc l'utilisation de nanoparticules de SiC de taille inférieure à 10 nm pour la préparation d'un médicament destiné au traitement du cancer.

Les nanoparticules de SiC sont chimiquement, thermiquement et mécaniquement stables. Par ailleurs, elles sont stables aux radiations (UVA, UVB et rayons X notamment). Elles sont non toxiques et faciles d'incorporation, notamment dans les cellules vivantes saines, ce qui permet de les utiliser comme marqueur fluorescent (Botsoa et al., Applied Physics Letters, 92, 173902, 2008).

Ainsi, les inventeurs ont constaté, après incorporation de nanoparticules de SiC, que celles-ci présentent un fort effet thérapeutique sur les cellules cancéreuses, sans toutefois affecter les cellules non cancéreuses.

En effet, les nanoparticules de SiC mises en oeuvre dans le cadre de la présente invention provoquent la mort des cellules cancéreuses humaines tout en permettant aux cellules humaines saines de survivre.

Selon un mode de réalisation préféré, les nanoparticules de SiC susmentionnées présentent une taille comprise de 0,5 nm à 10 nm.

Selon un mode de réalisation particulièrement préféré, la taille desdites nanoparticules est inférieure à 10 nm, de préférence inférieure à 7 nm, et préférentiellement inférieure à 5 nm.

Selon un mode de réalisation avantageux, la présente invention concerne des nanoparticules telles que définies ci-dessus pour leur utilisation dans le cadre du traitement du cancer, caractérisées en ce qu'elles sont sous forme d'une suspension.

De préférence, les nanoparticules selon la présente invention sont sous forme d'une suspension dans un tampon physiologique aqueux.

L'expression "tampon physiologique aqueux" désigne ici toute solution physiologique compatible pouvant être administrée à un être humain.

Parmi les tampons physiologiques adaptés, on peut notamment citer le sérum physiologique, les milieux de culture pour cellules *in vitro* tels que par exemple les milieux RPMI ou DMEM.

Dans le cadre de la présente invention, lesdites nanoparticules, sous forme d'une suspension, sont présentes à une concentration comprise de 0 à 20 g.L⁻¹ dans ladite suspension.

Selon un mode de réalisation particulier, les nanoparticules susmentionnées sont présentes dans ladite suspension à une concentration comprise de 0,5 g.L⁻¹ à 2 g.L⁻¹.

La présente invention concerne également les nanoparticules de SiC susmentionnées pour leur utilisation dans le cadre du traitement du cancer, caractérisées en ce que ces nanoparticules sont administrées par voie intraveineuse, par exemple sous forme d'une solution aqueuse.

Les nanoparticules susmentionnées peuvent également être administrées par injection locale par seringue.

Une autre voie d'administration appropriée de ces nanoparticules est la voie cutanée, par exemple en appliquant sur le site cancéreux à soigner une crème ou une lotion les contenant.

On peut également envisager d'administrer lesdites nanoparticules par voie orale par ingestion de comprimés ou de solutions les contenant.

Selon un mode de réalisation préféré, les nanoparticules utilisées dans le cadre de la présente invention sont susceptibles d'être obtenues selon un procédé de gravure d'un substrat de SiC, la gravure étant obtenue par attaque électrochimique du substrat de SiC.

L'expression "substrat de SiC" désigne, dans le cadre de la présente invention, tout composé chimique constitué des atomes de silicium (Si) et de carbone (C) liés chimiquement entre eux. La structure atomique du substrat de SiC (et donc des nanoparticules) peut être monocristalline, polycristalline ou amorphe. Le polytype du substrat de SiC peut être par exemple : 6H-SiC, 4H-SiC, 3C-SiC, 15R-SiC, 21R-SiC, 24R-SiC, 27R-SiC, etc. La composition chimique du substrat de SiC peut être par exemple : SiC ou tout autre composé exprimé par la formule SixC(1-x) avec 0<x<1. Le substrat de SiC peut contenir d'autres impuretés chimiques (par exemple : hydrogène, oxygène, azote, aluminium, phosphore, bore, béryllium, gallium, titane, chrome) dont la concentration (pour chaque impureté) ne dépassera pas 1% (c'est-à-dire inférieure à 1 %) de la concentration des atomes de Si et C.

Avantageusement, les nanoparticules sont obtenues par gravure d'un substrat de SiC massif.

Dans le cadre de la présente invention, l'expression "substrat massif" de SiC, désigne tout composé chimique constitué des atomes de silicium (Si) et de carbone (C) liés chimiquement entre eux et dont au moins une dimension linéaire de la taille (hauteur, largeur, longueur, diamètre, etc.) est supérieure à 1 mm.

De préférence, la gravure du substrat de SiC est obtenue par une attaque électrochimique du substrat de SiC. Par exemple, la gravure est obtenue par une attaque électrochimique durant laquelle le substrat de SiC se trouve en contact avec un électrolyte formé au moins à base d'acide (acide fluorhydrique, par exemple). Ce substrat est traversé par un courant électrique. Les paramètres de gravure tels que la densité du courant, la composition chimique, la concentration de l'électrolyte, la pression et la température ambiante seront choisis en fonction des besoins (vitesse de gravure, etc).

Selon un autre mode de réalisation préféré, les nanoparticules utilisées dans le cadre de la présente invention sont susceptibles d'être obtenues selon un procédé par ablation laser d'un substrat de SiC à travers un volume d'eau ou d'un autre solvant.

Cette méthode consiste à diriger un faisceau laser pulsé sur une cible (substrat de SiC ici) qui se trouve en contact avec un solvant. L'impact du faisceau provoque la dispersion du substrat SiC dans le solvant sous forme de petites nanoparticules. Cette méthode présente l'avantage de ne pas nécessiter l'utilisation d'acide fluorhydrique. De plus, il n'est pas nécessaire de rincer l'échantillon (à savoir que lors du rinçage on perd une quantité importante des nanoparticules formées).

Selon un autre mode de réalisation préféré, les nanoparticules utilisées dans le cadre de la présente invention sont susceptibles d'être obtenues selon un procédé par pyrolyse laser à partir par exemple d'un mélange de C₂H₂ et de SiH₄. Ces nanoparticules sont préparées comme décrit notamment dans les articles suivants : Leconte et al. (2007) J. Anal. Appl. Pyrolysis, 79, 465 et Herlin-Boime et al. (2004) J. Nanopart. Res., 6, 63.

De préférence, la présente invention concerne les nanoparticules de SiC susmentionnées pour leur utilisation dans le cadre du traitement de formes avancées de cancers avec métastases.

Tout type de cancer pourra être traité par les nanoparticules soit par administration intraveineuse soit par application locale. On peut notamment citer les cancers de la bouche ou de la gorge.

Enfin, la présente invention concerne également les nanoparticules de SiC susmentionnées pour leur utilisation dans le cadre du traitement du cancer, caractérisées en ce qu'elles sont administrées en association avec un autre médicament anti-cancéreux.

La présente invention concerne donc également les nanoparticules de SiC susmentionnées, en combinaison avec un médicament anti-cancéreux, pour leur utilisation dans le cadre du traitement du cancer.

Parmi les agents anti-cancéreux, on peut citer les médicaments altérant l'ADN, les antimétabolites, les inhibiteurs enzymatiques, les cytokines et les médicaments altérant le fuseau mitotique.

Parmi les médicaments altérant l'ADN, on peut citer les agents alkylants comme la chlométhine, les agents intercalants comme la daunorubicine, les inhibiteurs de la topoisomérase I et II comme l'irinotécan, le topotécan ou l'étoposide, les intermédiaires électrophiles comme les dérivés du platine tels que le cisplatine, le carboplatine et l'oxaliplatine.

Parmi les antimétabolites, on peut notamment citer le 5-fluoro-uracile ou la mercaptopurine.

Parmi les inhibiteurs enzymatiques, on peut notamment citer les inhibiteurs de la thymidylate synthase comme le raltitrexed, les inhibiteurs de la ribonucléide diphosphate réductase comme l'hydroxyurée ou encore les inhibiteurs de la dihydrofolate réductase comme le méthotréxate.

Parmi les cytokines, on peut notamment citer l'interféron alpha.

Parmi les médicaments altérant le fuseau mitotique, on peut notamment citer la vinblastine, la vincristine, la vindésine, la navelbine ou les taxanes comme le taxol, le paclitaxel ou le docétaxel.

Comme agents anti-cancéreux, on peut également citer les agents de chimiothérapie suivants : l'azathioprine, la capécitabine, la cytarabine, la floxuridine, la fludarabine, la gemcitabine, le méthotrexate, le pémétrexed, le busulfan, le chlorambucil, le cyclophosphamide, l'ifosfamide, le melphalan, la méchloréthamine, l'uramustine, la bléomycine, la doxorubicine, l'épirubicine, l'idarubicine, la mitomycine C et la mitoxantrone.

II a été démontré que les nanoparticules de SiC selon la présente invention ont des effets comparables aux molécules utilisées classiquement en tant qu'anticancéreux en terme d'efficacité.

Par ailleurs, dans le cadre de la présente invention, il a été constaté que l'association des nanoparticules selon l'invention et d'un agent anticancéreux classique présente une synergie en ce qui concerne l'effet thérapeutique. Ainsi, on peut remplacer le traitement classique via un agent anticancéreux par un traitement de type "cocktail" constitué d'un agent anticancéreux et des nanoparticules de l'invention. L'utilisation d'un tel "cocktail" permet, dans le cadre du traitement d'un cancer peu développé (début de la maladie), de diminuer la concentration de l'agent anticancéreux classique par rapport à la concentration de nanoparticules de SiC dans le "cocktail", ce qui entraîne l'obtention d'un effet thérapeutique identique (par rapport à la concentration de l'agent anticancéreux mise en oeuvre dans les traitements classiques connus à ce jour) mais la diminution des effets secondaires liés à l'utilisation de l'agent anticancéreux classique. Par contre, l'utilisation d'un tel "cocktail" permet, dans le cadre du traitement d'un cancer bien développé, en maintenant la concentration de l'agent anticancéreux par rapport aux traitements classiques, d'augmenter l'effet thérapeutique.

La présente invention concerne également les nanoparticules de SiC susmentionnées pour leur utilisation dans le cadre du traitement du cancer, caractérisées en ce que lesdites nanoparticules sont fonctionnalisées chimiquement. L'expression "nanoparticules fonctionnalisées" désigne ainsi des nanoparticules modifiées chimiquement, c'est-à-dire dont la surface a été modifiée par exemple par greffage de groupes chimiques appropriés. Ainsi, les nanoparticules de SiC susmentionnées peuvent être soumises à une fonctionnalisation chimique afin de modifier leur chimie de surface qui est notamment décrite en détail dans Alekseev et al. (Chemistry of Materials, 2007, vol. 19, 2189-2194). Cette fonctionnalisation permet d'adresser les nanoparticules dans des sites spécifiques des cellules.

La présente invention concerne également les nanoparticules susmentionnées pour leur utilisation dans le cadre du traitement du cancer, caractérisées en ce que au moins un médicament anti-cancéreux est greffé à la surface desdites nanoparticules.

Les nanoparticules ainsi modifiées permettent d'obtenir un adressage des médicaments anti-cancéreux vers les noyaux des cellules cancéreuses et donc d'améliorer l'efficacité du traitement, dans la mesure où les nanoparticules de SiC utilisées dans le cadre de la présente invention, une fois incorporées dans des cellules vivantes, pénètrent jusque dans le noyau desdites cellules (Botsoa et al., Applied Physics Letters, 92, 173902, 2008).

### FIGURES

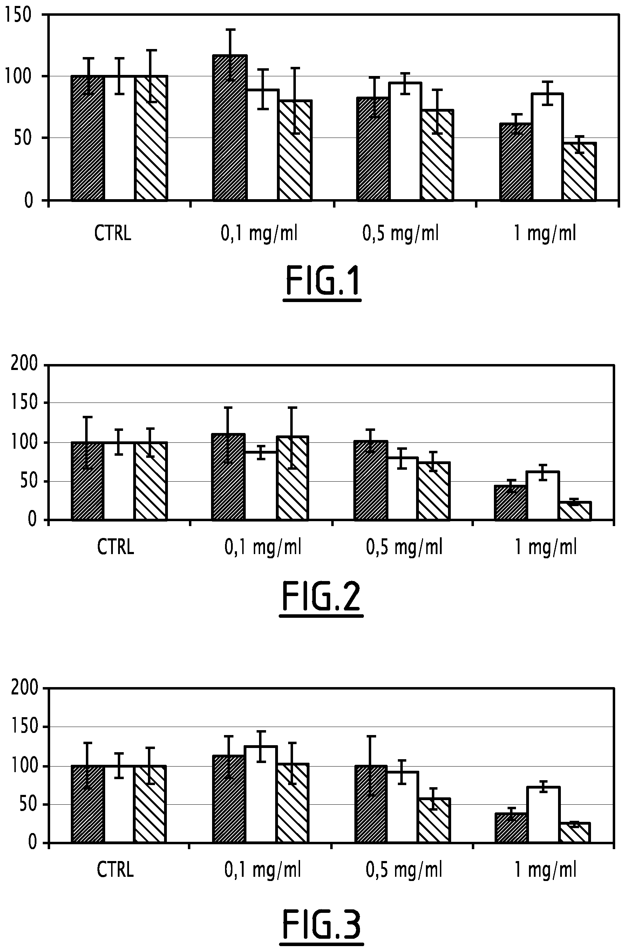

Les Figures 1, 2 et 3 représentent le pourcentage de survie cellulaire de différentes lignées cellulaires traitées par différentes concentrations de nanoparticules de SiC, et ce, respectivement, après 24, 48 ou 72 heures d'incubation. Les rectangles hachurés correspondent aux résultats pour la lignée cellulaire HSC (lignée cancéreuse humaine) ; les rectangles noirs correspondent aux résultats pour la lignée cellulaire AT84 (lignée cancéreuse murine) et les rectangles blancs correspondent aux résultats pour la lignée cellulaire SG (lignée cellulaire saine humaine immortalisée).

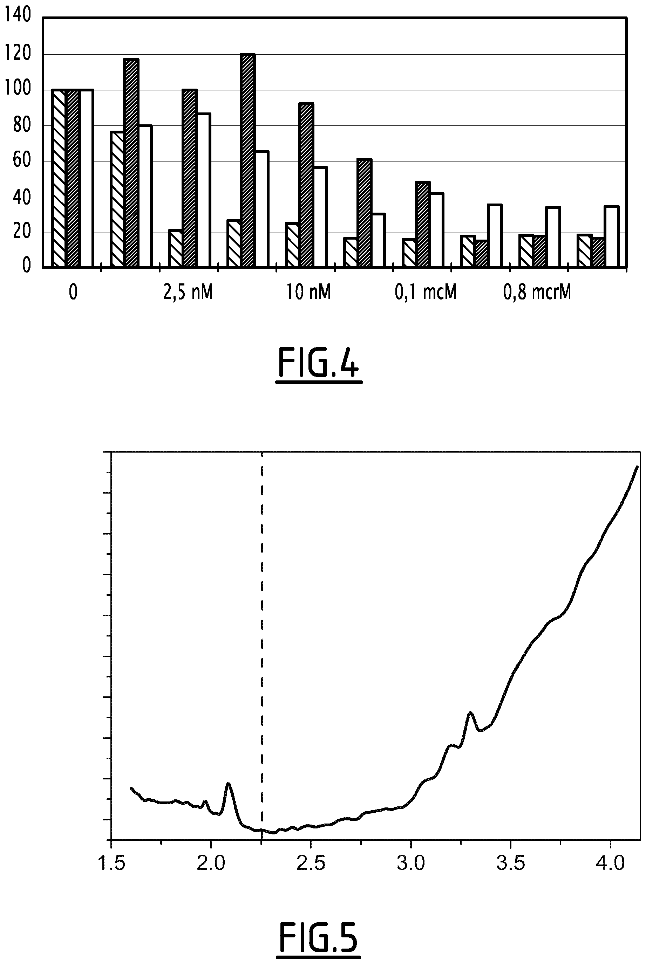

La Figure 4 représente l'effet d'une molécule anticancéreuse, à savoir le paclitaxel, sur différentes lignées cellulaires humaines après 72 heures de traitement. Le pourcentage de survie cellulaire est indiqué pour différentes concentrations de paclitaxel. Les rectangles hachurés correspondent aux résultats pour la lignée cellulaire HSC ; les rectangles noirs correspondent aux résultats pour la lignée cellulaire AT84 et les rectangles blancs correspondent aux résultats pour la lignée cellulaire SG.
La Figure 5 représente le spectre d'absorption de solutions de nanoparticules de SiC obtenues par ablation laser selon l'exemple 2 (absorption en unités arbitraires en fonction de l'énergie en eV). La ligne verticale en pointillés représente la valeur de E_{g} du substrat SiC initial.

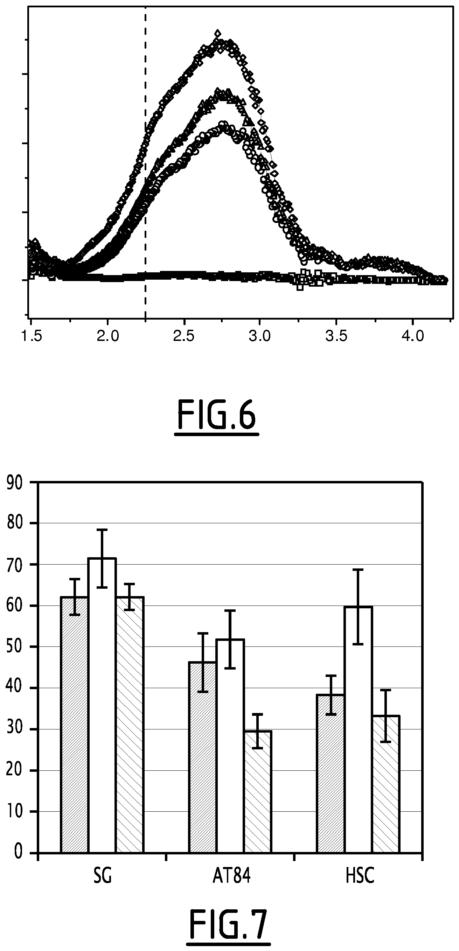

La Figure 6 représente les spectres de photoluminescence de solutions de nanoparticules de SiC dans de l'eau désionisée, obtenues par 3 puissances du laser (d'ablation)(signal de photoluminescence en unités arbitraires en fonction de l'énergie en eV)(T = 300 K et Eₑₓ = 5,08 eV). La courbe avec les carrés correspond au spectre de l'eau désionisée seule (sans nanoparticules), la courbe avec les cercles correspond à une puissance du laser de 115 mW, la courbe avec les triangles correspond à une puissance du laser de 230 mW et la courbe avec les losanges correspond à une puissance du laser de 460 mW. La ligne verticale en pointillés représente la valeur de E_{g} du substrat SiC initial.
La Figure 7 représente les effets des nanoparticules de la présente invention (rectangles grisés), du cisplatine (rectangles blancs) et de la combinaison de ces nanoparticules et du cisplatine (rectangles hachurés), sur différentes lignées cellulaires humaines après 72 heures de traitement. Les trois rectangles de gauche correspondent aux résultats pour la lignée cellulaire SG ; les trois rectangles du milieu correspondent aux résultats pour la lignée cellulaire AT84 et les trois rectangles de droite correspondent aux résultats pour la lignée cellulaire HSC.

### PARTIE EXPÉRIMENTALE

### Exemple 1 - Préparation de nanoparticules par gravure électrochimique et propriétés des nanoparticules ainsi obtenues

### Description des essais effectués

Les nanoparticules de carbure de silicium (SiC) pénètrent dans les cellules animales. Elles se fixent d'abord sur des éléments présents dans le cytoplasme des cellules puis elles se concentrent dans le noyau des cellules.

On a testé l'effet d'une exposition de plusieurs types cellulaires à différentes concentrations de nanoparticules de SiC.

Les nanoparticules ont été produites par anodisation électrochimique à partir d'un wafer de 3C-SiC polycristalline à faible résistivité de 1 cm de rayon. Le procédé de gravure a duré pendant trois heures sous illumination UV, à un courant d'intensité 25 mA/cm², en utilisant une dilution de 1:1 HF 50% éthanol comme électrolyte. Après gravure, un réseau très poreux de nanocristaux interconnectés de 3C-SiC est formé. La couche ultra-poreuse est séchée naturellement à l'air ambiant. La nano-poudre de 3C-SiC obtenue par broyage de la couche nano-poreuse est mise en suspension dans une solution de tampon Krebs. Cette suspension est centrifugée à 5000xg pendant trois minutes afin de sédimenter les gros cristaux insolubles. La partie supérieure contenant les nanoparticules très petites (inférieures à 10 nm) en suspension uniforme est utilisée pour les essais.

La survie cellulaire a été mesurée à l'aide d'une méthode colorimétrique au bromure de 3-(4,5-diméthylthiazol-2-yl)-2,5-diphényltétrazolium (MTT). Cette molécule de couleur jaune est réduite par les enzymes déshydrogénases mitochondriales en sel de couleur bleu. Ceci permet de quantifier la viabilité cellulaire en réponse à un traitement. En effet, plus le nombre de cellules est élevé, plus elles transforment de MTT et donc plus la coloration bleue est intense.

La survie des cellules a été mesurée avant et après exposition à différentes concentrations de nanoparticules de SiC (0,1 - 0,5 - 1 mg/mL dans une suspension de milieu RPMI 10% FCS).

Les cellules ont été cultivées sur des plaques de 96 puits en présence de nanoparticules de SiC pendant 18 heures et ensuite les milieux ont été remplacés par des milieux de culture standard sans nanoparticules (milieu RPMI 10% FCS, Sigma).

La viabilité des cellules est mesurée après différentes durées (24, 48 et 72 heures). Les cultures de cellules ont ensuite été utilisées pour réaliser le test au MTT.

La viabilité cellulaire a été mesurée par la différence de densité optique aux longueurs d'onde 450 et 504 nm, en utilisant un spectrophotomètre relié à un système informatique (Power Wave, Bio-Tek Instrument Inc.). L'absorbance mesurée est alors directement proportionnelle au nombre de cellules vivantes. Les résultats ont été exprimés en pourcentage de survie mesuré dans les conditions initiales avant l'exposition des cellules aux nanoparticules de SiC.

### Résultats

Les expérimentations ont été effectuées sur trois lignées de cellules :
- une lignée de cellules cancéreuses humaines (HSC) (lignée cellulaire de carcinome squameux humain HSC-2 dérivée de la cavité orale) ;
- une lignée de cellules humaines saines immortalisées (SG) (lignée cellulaire humaine immortalisée de l'épithélium gingival, don du Dr. Babich) ; et
- une lignée de cellules cancéreuses de souris (AT84) (lignée cellulaire dérivée d'un carcinome squameux oral spontané de souris C3H, don du Dr. Shillitoe).

La viabilité des cellules est mesurée après différentes durées (24, 48 et 72 heures). Les résultats correspondants sont indiqués respectivement dans les Figures 1, 2 et 3.

En observant les résultats, on note en comparant les lignées HSC (cancéreuses humaines) et SG (humaines immortalisées) que la survie des cellules cancéreuses humaines est significativement diminuée dès 24 heures pour la dose 0,5 mg/mL. Cet effet significatif est seulement présent chez les cellules des lignées cancéreuses.

Pour une concentration supérieure, l'effet inhibiteur des nanoparticules augmente. A 1 mg/mL, la viabilité des cellules humaines immortalisées est légèrement diminuée. II existe une différence significative de l'effet des nanoparticules entre les cellules saines et les cellules cancéreuses. Les effets sont d'autant plus importants quand la durée de l'exposition des cellules augmente (voir 48 et 72 heures pour la dose 0,5 mg/mL sur les cellules cancéreuses humaines). La survie des cellules HSC est de 20 % en réponse à 1 mg/mL après 72 heures, alors que celle des cellules SG (humaines immortalisées) est de 70%.

Les effets sur la lignée AT84 (cellules cancéreuses de souris) sont très significatifs pour la concentration 1 mg/mL (p<0,001) dès 24 heures et sont confirmés après 48 et 72 heures. Ceci permet de conclure que c'est la cancérogénicité des cellules qui est responsable d'une plus grande sensibilité aux nanoparticules de SiC.

Les résultats obtenus sur des lignées de cellules saines et cancéreuses en culture montrent que les nanoparticules de SiC diminuent spécifiquement le nombre de cellules cancéreuses alors que les cellules saines sont très significativement moins affectées par les nanoparticules de SiC.

La comparaison des effets des nanoparticules avec une molécule anticancéreuse (le paclitaxel) montre que les pourcentages d'inhibition de la survie cellulaire sont du même ordre (Figure 4).

II faut souligner que le traitement avec les nanoparticules n'a duré que 18 heures et que les cellules ont ensuite été rincées, alors que les médicaments anti-cancéreux ont été appliqués aux cultures cellulaires pendant les 72 heures de l'étude.

La différence de l'effet des nanoparticules entre les cellules cancéreuses et non cancéreuses met en évidence un effet sélectif des nanoparticules de SiC sur l'inhibition des cellules cancéreuses.

### Exemple 2 - Préparation de nanoparticules par ablation laser et propriétés des nanoparticules ainsi obtenues

Des nanoparticules de SiC ont été préparées selon le procédé par ablation laser en suivant le protocole tel que décrit dans les publications suivantes : A. V. Kabashin et al., J. Photochem. Photobiol. A (2006), 182 330, A. V. Kabashin et al. (2003), J. Appl. Phys., 94, 7941 et S. Barcikowski et al. (2007) Appl. Phys. A, 87, 47.

On a ainsi obtenu des nanoparticules de petite taille, à savoir de taille inférieure à 10 nm.

D'après la Figure 5, on constate que le seuil d'absorption de l'ensemble des nanoparticules obtenues se trouve vers 3 eV, ce qui indique que la plupart des nanoparticules présente une taille inférieure à 5,4 nm.

Par ailleurs, d'après la Figure 6, les intensités importantes de photoluminescence qui correspondent aux énergies supérieures à 2,25 eV confirment que les tailles des nanoparticules sont inférieures à 5,4 nm (diamètre de Bohr de l'exciton dans 3C-SiC).

### Exemple 3 - Association des nanoparticules selon l'invention avec des agents anticancéreux

Des essais ont été effectués afin de comparer les effets des nanoparticules de SiC sur la survie cellulaire avec les effets du cisplatine (anticancéreux de référence). D'autres essais ont également été effectués afin de comparer les effets de la combinaison des nanoparticules de SiC et du cisplatine sur la survie cellulaire avec les effets, d'une part, du cisplatine seul, et d'autre part, des nanoparticules de SiC seules.

Les résultats sont indiqués dans la Figure 7.

La survie cellulaire a été mesurée à l'aide d'une méthode colorimétrique au bromure de 3-(4,5-diméthylthiazol-2-yl)-2,5-diphényltétrazolium (MTT) telle que décrite ci-dessus dans l'exemple 1.

La survie des cellules a été mesurée après exposition aux nanoparticules de SiC selon l'exemple 1 (0,5 mg/mL), à du cisplatine (1,5 µM) et au mélange nanoparticules de SiC (0,5 mg/mL) et cisplatine (1,5 µM).

Les cellules ont été cultivées sur des plaques de 96 puits en présence de nanoparticules de SiC.

La viabilité des cellules est mesurée après 72 heures. Les cultures de cellules ont ensuite été utilisées pour réaliser le test au MTT.

La viabilité cellulaire a été mesurée par la différence de densité optique comme décrit dans l'exemple 1.

Les résultats indiqués sont normalisés par rapport au contrôle (cellules non traitées).

D'après la Figure 7, on constate, comme déjà indiqué dans l'exemple 1, que les nanoparticules de SiC de l'invention affectent moins la survie des cellules saines (lignée SG) que les cellules cancéreuses (lignées AT84 et HSC).

Pour les lignées de cellules cancéreuses, l'addition de nanoparticules de SiC permet d'augmenter l'effet du cisplatine sur la survie cellulaire, ce qui démontre la synergie.

On observe que la survie cellulaire des cellules mises en culture avec le cocktail cisplatine + nanoparticules de SiC est inférieure à celle des cellules mises en culture avec le cisplatine seul.

## Revendications

1. Nanoparticules de SiC pour leur utilisation dans le cadre du traitement du cancer, **caractérisées en ce que** leur taille est inférieure à 10 nm.

2. Nanoparticules pour leur utilisation selon la revendication 1, **caractérisées en ce que** leur taille est comprise de 0,5 nm à 10 nm.

3. Nanoparticules pour leur utilisation selon l'une quelconque des revendications 1 ou 2, **caractérisées en ce que** leur taille est inférieure à 7 nm.

4. Nanoparticules pour leur utilisation selon la revendication 1 ou 2, **caractérisées en ce que** leur taille est inférieure à 5 nm.

5. Nanoparticules pour leur utilisation selon l'une quelconque des revendications 1 à 4, **caractérisées en ce qu'**elles sont sous forme d'une suspension dans un tampon physiologique aqueux.

6. Nanoparticules pour leur utilisation selon la revendication 5, **caractérisées en ce qu'**elles sont présentes à une concentration comprise de 0 à 20 g.L⁻¹.

7. Nanoparticules pour leur utilisation selon la revendication 5, **caractérisées en ce qu'**elles sont présentes à une concentration comprise de 0,5 à 2 g.L⁻¹.

8. Nanoparticules pour leur utilisation selon la revendication 7, **caractérisées en ce qu'**elles sont administrées par voie intraveineuse, par injection locale, par voie cutanée ou par voie orale.

9. Nanoparticules pour leur utilisation selon l'une quelconque des revendications 1 à 8, **caractérisées en ce qu'**elles sont susceptibles d'être obtenues selon un procédé par ablation laser d'un substrat de SiC ou selon un procédé de pyrolyse laser ou selon un procédé de gravure d'un substrat de SiC, la gravure étant obtenue par attaque électrochimique du substrat de SiC.

10. Nanoparticules pour leur utilisation selon l'une quelconque des revendications 1 à 9, **caractérisées en ce qu'**elles sont utilisées pour le traitement de formes avancées de cancers avec métastases.

11. Nanoparticules pour leur utilisation selon l'une quelconque des revendications 1 à 10, **caractérisées en ce qu'**un médicament anti-cancéreux est greffé à leur surface.

12. Nanoparticules pour leur utilisation selon l'une quelconque des revendications 1 à 10, **caractérisées en ce qu'**elles sont administrées en association avec un médicament anti-cancéreux.

## Patentansprüche

1. SiC-Nanopartikel für ihre Verwendung im Rahmen der Behandlung von Krebs, **dadurch gekennzeichnet, dass** ihre Größe kleiner als 10 nm ist.

2. Nanopartikel für ihre Verwendung gemäß Anspruch 1, **dadurch gekennzeichnet, dass** ihre Größe 0,5 nm bis 10 nm beträgt.

3. Nanopartikel für ihre Verwendung gemäß irgendeinem der Ansprüche 1 oder 2, **dadurch gekennzeichnet, dass** ihre Größe kleiner als 7 nm ist.

4. Nanopartikel für ihre Verwendung gemäß Anspruch 1 oder 2, **dadurch gekennzeichnet, dass** ihre Größe kleiner als 5 nm ist.

5. Nanopartikel für ihre Verwendung gemäß irgendeinem der Ansprüche 1 bis 4, **dadurch gekennzeichnet, dass** sie in Form einer Suspension in einem wässrigen physiologischen Puffer vorliegen.

6. Nanopartikel für ihre Verwendung gemäß Anspruch 5, **dadurch gekennzeichnet, dass** sie in einer Konzentration vorliegen, die 0 g.L⁻¹ bis 20 g.L⁻¹ beträgt.

7. Nanopartikel für ihre Verwendung gemäß Anspruch 5, **dadurch gekennzeichnet, dass** sie in einer Konzentration vorliegen, die 0,5 g.L⁻¹ bis 2 g.L⁻¹ beträgt.

8. Nanopartikel für ihre Verwendung gemäß Anspruch 7, **dadurch gekennzeichnet, dass** sie intravenös, durch lokale Injektion, perkutan oder oral verabreicht werden.

9. Nanopartikel für ihre Verwendung gemäß irgendeinem der Ansprüche 1 bis 8, **dadurch gekennzeichnet, dass** sie geeignet sind, gemäß einem Verfahren durch Laserablation eines SiC-Substrats oder gemäß einem Laserpyrolyseverfahren oder gemäß einem Verfahren zum Ätzen eines SiC-Substrats erhalten werden, wobei das Ätzen durch elektrochemisches Angreifen des SiC-Substrats erhalten wird.

10. Nanopartikel für ihre Verwendung gemäß irgendeinem der Ansprüche 1 bis 9, **dadurch gekennzeichnet, dass** sie für die Behandlung von fortgeschrittenen Krebsformen mit Metastasen verwendet werden.

11. Nanopartikel für ihre Verwendung gemäß irgendeinem der Ansprüche 1 bis 10, **dadurch gekennzeichnet, dass** ein Krebsmedikament auf ihre Oberfläche gepfropft wird.

12. Nanopartikel für ihre Verwendung gemäß irgendeinem der Ansprüche 1 bis 10, **dadurch gekennzeichnet, dass** sie in Verbindung mit einem Krebsmedikament verabreicht werden.

## Claims

1. SiC nanoparticles for use thereof in the treatment of cancer, **characterized in that** their size is less than 10 nm.

2. The nanoparticles for use thereof according to claim 1, **characterized in that** they have a size of between 0.5 nm and 10 nm.

3. The nanoparticles for use thereof according to either of claims 1 or 2, **characterized in that** they have a size of less than 7 nm.

4. The nanoparticles for use thereof according to either of claims 1 or 2, **characterized in that** they have a size of less than 5 nm.

5. The nanoparticles for use thereof according to any of claims 1 to 4, **characterized in that** they are in the form of a suspension in an aqueous physiological buffer.

6. The nanoparticles for use thereof according to claim 5, **characterized in that** they are present at a concentration of from 0 to 20 g.L⁻¹.

7. The nanoparticles for use thereof according to claim 5, **characterized in that** they are present at a concentration of from 0.5 to 2 g.L⁻¹.

8. The nanoparticles for use thereof according to claim 7, **characterized in that** they are administered via intravenous route, via local injection, via cutaneous route or via oral route.

9. The nanoparticles for use thereof according to any of claims 1 to 8, **characterized in that** they are able to be obtained using a method for laser ablation of a SiC substrate, or using a laser pyrolsyis method, or using a SiC substrate etching method, the etching being obtained by electrochemical attack of the SiC substrate.

10. The nanoparticles for use thereof according to any of claims 1 to 9, **characterized in that** they are used for the treatment of advanced forms of cancer with metastasis.

11. The nanoparticles according to any of claims 1 to 10, **characterized in that** an anticancer drug is grafted on their surface.

12. The nanoparticles according to any of claims 1 to 10, **characterized in that** they are administered in combination with an anticancer drug.
